# EUROPEAN PATENT APPLICATION

(11) **EP 2 241 257 A2**
(43) Date of publication of application: **20.10.2010**
(21) Application number: 10157873.0
(22) Date of filing: 26.03.2010
(51) Int. Cl.: A61B 8/08, A61B 10/02, A61B 10/00

(54) **Integrated-type ultrasonic diagnostic apparatus with separable biopsy device**

(30) Priority: 14.04.2009 KR 20090032266
(71) Applicant: Medison Co., Ltd., Kangwon-do 250-870 (KR)
(72) Inventor: Kim, Seong Rae, Gyeonggi-do (KR); Hyun, Dong Gyu, Gyeonggi-do (KR); Kim, Chul An, Gyeonggi-do (KR); Park, Hee Jung, Seoul (KR)
(74) Representative: Schmid, Wolfgang

(57) **Abstract**

A separable biopsy device integrated-type ultrasonic diagnostic apparatus is disclosed. The separable biopsy device integrated-type ultrasonic diagnostic apparatus includes an ultrasonic diagnostic apparatus (100) exposing one side of a diagnosis target (10) and examining the diagnosis target (10) at the other side of the diagnosis target (10); and a biopsy device (200) located at the exposed side of the diagnosis target (10) and detachably coupled to the ultrasonic diagnostic apparatus (100). The apparatus can perform ultrasound diagnosis and biopsy together, thereby providing the convenience of allowing the ultrasound diagnosis and the biopsy to be performed with a single apparatus, and can perform a biopsy based on ultrasound images obtained using the ultrasonic diagnostic apparatus (100) without changing a position of a diagnosis target (10), enabling precise determination as to a position to be biopsied, that is, a target position into which the needle of the biopsy device (200) will be inserted.

## Description

### BACKGROUND OF THE INVENTION

### 1. Field of the Invention

The present invention relates to an ultrasonic diagnostic apparatus and, more particularly, to a separable biopsy device integrated-type ultrasonic diagnostic apparatus that can perform ultrasound diagnosis and biopsy together.

### 2. Description of the Related Art

Breast cancer is not only the most common type of cancer in the West, but also has a very high incidence rate together with cervical cancer and stomach cancer among Korean women. Primary diagnosis of breast cancer is generally performed using an X-ray imaging system. The X-ray imagine system is very convenient for diagnosis and thus has been widely used in the art. However, the X-ray imaging system has a very low diagnosis rate for dense tissue of the breast.

The X-ray imaging system has significantly low effectiveness and a very high misdiagnosis rate in diagnosis of breast cancer, particularly for Korean women who generally have much denser breast tissue than Western women.

Recently, an ultrasonic diagnostic apparatus has been employed instead of the X-ray imaging system. The ultrasonic diagnostic apparatus does not provide a danger of radiation exposure and is capable of processing images of a diagnosis target such as three-dimensional images and detecting small cancers of about 2∼3 mm.

The ultrasonic diagnostic apparatuses for diagnosis of breast cancers are generally classified into a supine type ultrasonic diagnostic apparatus configured to diagnose an examinee lying face up, a prone type ultrasonic diagnostic apparatus configured to diagnose an examinee lying face down, and an upright type ultrasonic diagnostic apparatus configured to diagnose an examinee standing or sitting therein.

Since the supine type and prone type ultrasonic diagnostic apparatuses require an examinee to lie face up or face down during diagnosis, they occupy a large installation space and entail a very complicated diagnosis procedure, thereby causing low diagnosis efficiency. Furthermore, in these types of ultrasonic diagnostic apparatuses, the examinee is diagnosed in an inconvenient posture and is thus likely to suffer fatigue during diagnosis.

Since the upright type ultrasonic diagnostic apparatus allows an examinee to stand or sit during diagnosis, it occupies a smaller installation space and provides a more convenient diagnosis procedure than the other types of ultrasonic diagnostic apparatuses.

The upright type ultrasonic diagnostic apparatus is provided with a compression rack that includes probes and performs diagnosis of an examinee in a standing or sitting posture while compressing a diagnosis target of the examinee up and down or right and left.

On the other hand, when a portion of the diagnosis target is suspected of having a tumor according to diagnosis of the ultrasonic diagnostic apparatus, the portion can be biopsied for more accurate diagnosis as to the existence of the tumor. The biopsy may be performed using a biopsy device that includes a needle inserted into the target to extract internal tissue from the diagnosis target.

As such, since the upright type ultrasonic diagnostic apparatus described above cannot perform a biopsy, the biopsy must be performed using a separate biopsy device independent of the ultrasound diagnosis in the case where the biopsy must be performed as a follow-up to the ultrasound diagnosis. Accordingly, the separate biopsy device is inconveniently provided independent of the ultrasonic diagnostic apparatus and the biopsy and the ultrasound diagnosis are separately performed, thereby causing difficulty in precise determination of a target position into which the needle will be inserted. Therefore, there is a need to solve such a problem.

### SUMMARY OF THE INVENTION

The present invention is conceived to solve the problem of the related art, and an aspect of the invention is to provide an improved separable biopsy device integrated-type ultrasonic diagnostic apparatus that can perform ultrasound diagnosis and biopsy together and can make precise determination as to a target position to be biopsied, thereby enabling efficient biopsy.

In accordance with one aspect of the invention, a separable biopsy device integrated-type ultrasonic diagnostic apparatus includes: an ultrasonic diagnostic apparatus exposing one side of a diagnosis target and examining the diagnosis target at the other side of the diagnosis target; and a biopsy device located at the exposed side of the diagnosis target and detachably coupled to the ultrasonic diagnostic apparatus.

The separable biopsy device integrated-type ultrasonic diagnostic apparatus may further include: a first coupling part provided to the ultrasonic diagnostic apparatus; and a second coupling part provided to the biopsy device to be detachably coupled to the first coupling part.

One of the first and second coupling parts may be formed with a coupling groove, and the other may be formed with a coupling protrusion inserted into the coupling groove.

The separable biopsy device integrated-type ultrasonic diagnostic apparatus may further include a securing part that secures coupling between the first coupling part and the second coupling part.

The biopsy device may include: a needle collecting a tissue sample from the diagnosis target; a biopsy kit having a needle guide guiding a movement path of the needle; and a biopsy kit-guide coupled with the biopsy kit and guiding a movement path of the biopsy kit.

The biopsy kit-guide may include a first guide member disposed in a first direction to guide the biopsy kit in the first direction; and a second guide member disposed in a different direction from that of the first guide member to guide the biopsy kit in a second direction.

The ultrasonic diagnostic apparatus may include: a housing exposing one side of the diagnosis target while supporting the diagnosis target so as to correct a shape of the diagnosis target; an oblique part obliquely formed on the housing to support the diagnosis target; and a probe unit disposed inside the housing to examine the diagnosis target.

The oblique part may have an upward slope corresponding to the shape of the diagnosis target from a lower side of the oblique part to an upper side thereof.

The diagnosis target may be a breast of an examinee, the oblique part may have a length supporting both breasts of the examinee, and the probe unit may move along a path including a curved path to examine both breasts of the examinee through a single examination operation.

The ultrasonic diagnostic apparatus may further include a lift part raising or lowering the housing.

The ultrasonic diagnostic apparatus may further include an oblique movement part obliquely moving the housing to approach or move away from the diagnosis target.

The ultrasonic diagnostic apparatus may be an upright type ultrasonic diagnostic apparatus.

According to the invention, the separable biopsy device integrated-type ultrasonic diagnosis apparatus can perform ultrasound diagnosis and biopsy together, thereby providing the convenience of allowing the ultrasound diagnosis and the biopsy to be performed with a single apparatus.

Further, the separable biopsy device integrated-type ultrasonic diagnostic apparatus can perform a biopsy based on ultrasound images obtained using the ultrasonic diagnostic apparatus without changing a position of a diagnosis target, enabling precise determination as to a position to be biopsied, that is, a target position into which the needle of the biopsy device will be inserted.

Further, the separable biopsy device integrated-type ultrasonic diagnostic apparatus allows the biopsy device to be separated and stored separately from the ultrasonic diagnostic apparatus when not in use, thereby reducing a space occupied by the entire apparatus and preventing damage of the biopsy device due to disuse of the biopsy device for a long period of time in a state of being coupled to the ultrasonic diagnostic apparatus.

### BRIEF DESCRIPTION OF THE DRAWINGS

The above and other aspects, features and advantages of the invention will become apparent from the following description of embodiments given in conjunction with the accompanying drawings, in which:
- Fig. 1: is a perspective view of a separable biopsy device integrated-type ultrasonic diagnostic apparatus in accordance with an embodiment of the present invention;
- Fig. 2: is a view of one exemplary use of the separable biopsy device integrated-type ultrasonic diagnostic apparatus in accordance with the embodiment of the present invention;
- Fig. 3: is a cross-sectional view taken along line A-A of Fig. 2;
- Fig. 4: is a cross-sectional view taken along line B-B of Fig. 2;
- Fig. 5: is a side view of an oblique movement part of the ultrasonic diagnostic apparatus in accordance with the embodiment of the present invention;
- Figs. 6 and 7: are side views of a lift part of the ultrasonic diagnostic apparatus in accordance with the embodiment of the present invention;
- Fig. 8: is a view of another exemplary use of the ultrasonic diagnostic apparatus in accordance with the embodiment of the present invention;
- Fig. 9: is an exploded perspective view of the separable biopsy device integrated-type ultrasonic diagnostic apparatus in accordance with the embodiment of the present invention;
- Fig. 10: is a view of a coupling structure between the biopsy device and the ultrasonic diagnostic apparatus shown in Fig. 9;
- Fig. 11: is a plan view of the biopsy device shown in Fig. 9;
- Figs. 12 and 13: show an operation of the biopsy device shown in Fig. 11;
- Fig. 14: is a cross-sectional view taken along line C-C of Fig. 13; and
- Figs. 15 and 16: show an operation of a biopsy kit shown in Fig. 14.

### DETAILED DESCRIPTION OF THE EMBODIMENT

Embodiments of the invention will now be described in detail with reference to the accompanying drawings. It should be noted that the drawings are not to precise scale and may be exaggerated in thickness of lines or size of components for descriptive convenience and clarity only. Furthermore, terms used herein are defmed by taking functions of the invention into account and can be changed according to the custom or intention of users or operators. Therefore, definition of the terms should be made according to overall disclosures set forth herein.

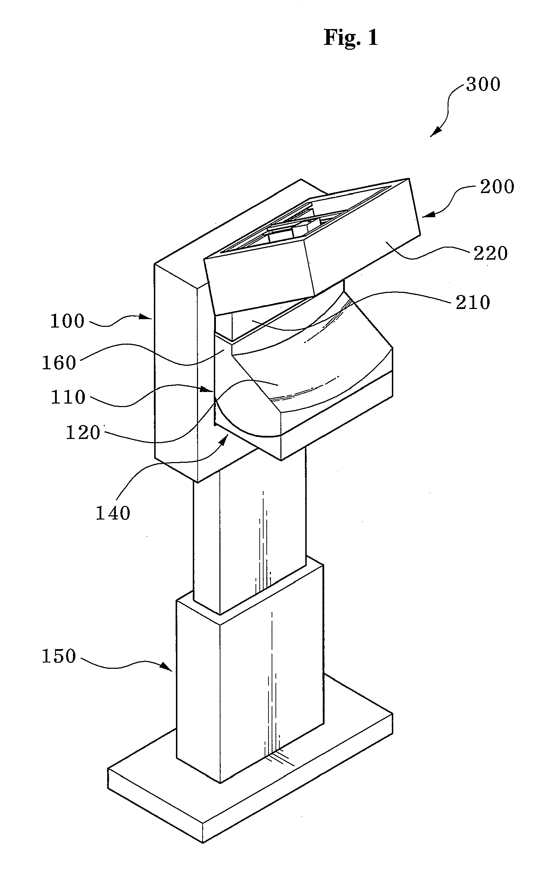

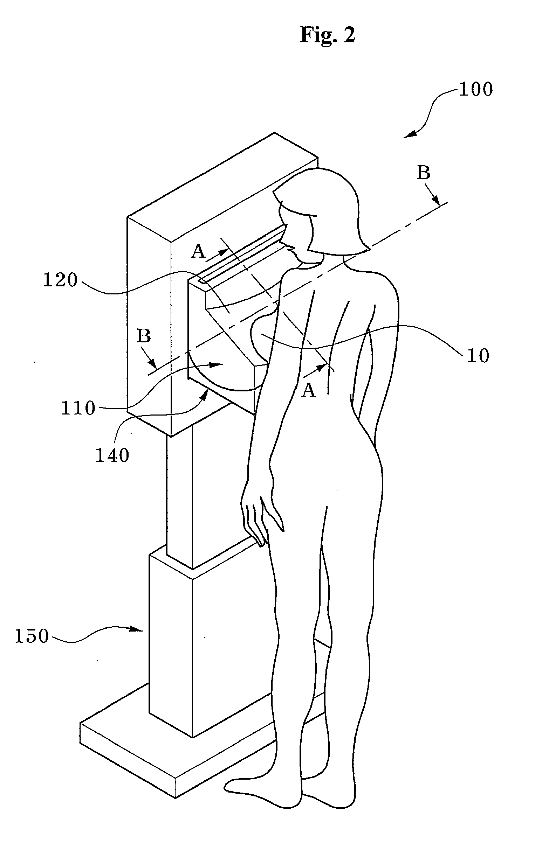

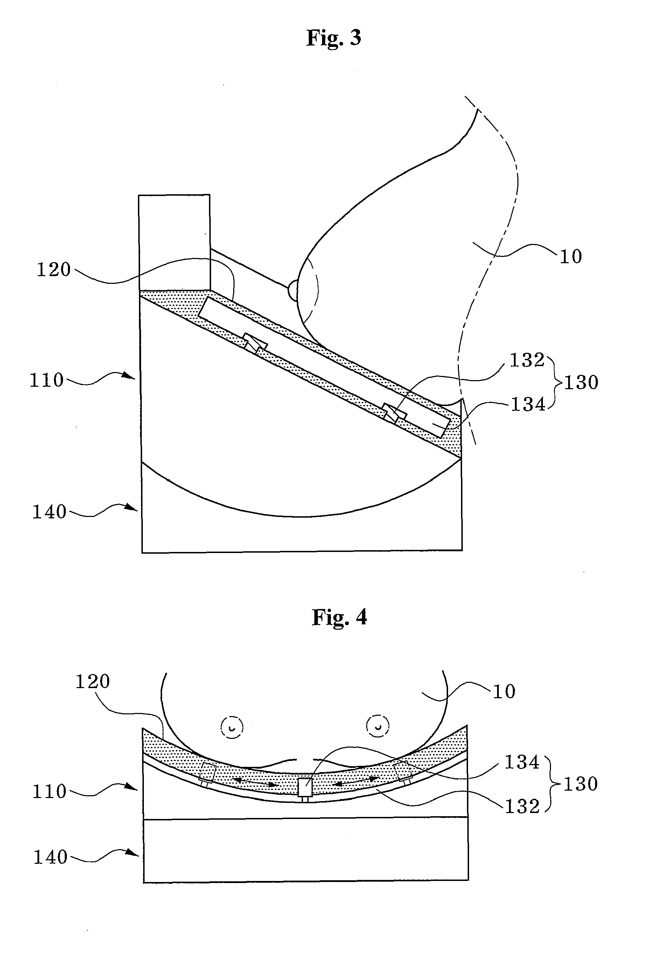

Fig. 1 is a perspective view of a separable biopsy device integrated-type ultrasonic diagnostic apparatus in accordance with an embodiment of the invention, Fig. 2 is a view of one exemplary use of the separable biopsy device integrated-type ultrasonic diagnostic apparatus in accordance with the embodiment, Fig. 3 is a cross-sectional view taken along line A-A of Fig. 2, and Fig. 4 is a cross-sectional view taken along line B-B of Fig. 2.

First, referring to Fig. 1, a separable biopsy device integrated-type ultrasonic diagnostic apparatus 300 according to one embodiment includes an ultrasonic diagnostic apparatus 100 and a biopsy device 200.

Referring to Figs. 1 to 4, the ultrasonic diagnostic apparatus 100 is an upright type breast ultrasonic diagnostic apparatus that can carry out ultrasound diagnosis for a diagnosis target of an examinee in a standing or sitting posture. The ultrasonic diagnostic apparatus 100 includes a housing 110, an oblique part 120, and a probe unit 130.

The housing 110 supports a diagnosis target 10. The housing 110 may be filled with a fluid that facilitates transmission of ultrasound waves. The fluid in the housing 110 may be oil or the like. In the housing 110, an ultrasound probe 134 of the probe unit 130 is immersed in the fluid.

In this embodiment, the housing 110 supports the diagnostic target 10 and is configured to expose one side, for example, an upper side, of the diagnostic target 10. As such, the housing 110 exposes the upper side of the diagnostic target 10 to secure a visual field of an operator so that the operator can check the position and state of the diagnosis target with the naked eye from above the upper side of the diagnosis target 10.

Further, the housing 110 supports the diagnosis target 10 so as to "maintain" the shape of the diagnosis target 10 while exposing the one side of the diagnosis target 10. Herein, the term "maintain" means that the housing 110 supports only one side of the diagnosis target 10 and does not compress the diagnosis target 10 to such a degree that an examinee experiences discomfort.

According to this embodiment, the diagnosis target 10 is illustrated as being the breast of a person. The diagnosis target 10 such as the breast is likely to be deformed upon compression.

In this embodiment, the housing 110 supports only the other side of the diagnosis target 10, for example, a lower side of the diagnosis target 10, thereby supporting the diagnosis target 10 without excessive compression of the diagnosis target 10. As a result, the diagnosis target 10 can be supported by the housing 110 without undergoing substantial shape change.

The oblique part 120 is obliquely formed on the housing 110 to support the diagnosis target 10. The oblique part 120 will directly contact the diagnosis target 10 while supporting the diagnosis target 10. The oblique part 120 has an upward slope corresponding to the shape of the diagnosis target 10 from a lower side of the oblique part 120 to an upper side thereof.

The oblique part 120 may have a length capable of supporting the diagnosis target 10, for example, both breasts of an examinee. Further, the oblique part 120 has a "linear shape" cross-section orthogonal to a longitudinal direction of the oblique part 120 (see Fig. 3) and a "curved shape" cross-section in a direction in which the probe unit 130 described below moves, that is, in the longitudinal direction of the oblique part 120 (see Fig. 4).

Herein, the "linear shape" cross-section of the oblique part 120 includes not only a completely linear shape but also a substantially linear shape, the overall shape of which approaches the linear shape. Further, the "curved shape" cross-section in the longitudinal direction of the oblique part 120 may be a curved shape similar to the shape of the diagnosis target 10, which will come into contact with the oblique part 120.

The probe unit 130 is disposed inside the housing 110. The probe unit 130 is movably disposed inside the housing 110 to examine the diagnosis target 10.

Further, the probe unit 130 may be obliquely disposed corresponding to the oblique part 120 and move along a path including a curved path defined in the longitudinal direction of the oblique part 120 so as to examine both breasts of an examinee supported on the oblique part 120 through a single diagnosis operation. The probe unit 130 includes a guide member 132 and an ultrasound probe 134.

The guide member 132 is formed to include a curved shape. The guide member 132 may be obliquely disposed corresponding to the oblique part 120 and may be formed to include a curved shape corresponding to the longitudinal shape of the oblique part 120.

The ultrasound probe 134 is coupled to the guide member 132 and moved thereon. The ultrasound probe 134 is provided with a transducer (not shown), which transmits an ultrasound signal to the diagnosis target 10 and receives the ultrasound echo-signal reflected from the diagnosis target 10, and reciprocates on the rear side of the oblique part 120 along a path defmed by the guide member 132.

The ultrasound probe 134 examines the diagnosis target 10 by sending an ultrasound signal to the diagnosis target 10 and receiving the ultrasound echo-signal reflected from the diagnosis target 10, in which the ultrasound signal sent from the ultrasound probe 134 or reflected from the diagnosis target 10 is transmitted to the diagnosis target or the ultrasound probe 134 via the fluid having the ultrasound probe 134 immersed therein.

In this embodiment, the probe unit 130 may include a single or plurality of ultrasound probes 134.

When the probe 130 includes a single ultrasound probe 134, the ultrasound probe 134 of the probe 130 may have a width capable of examining the diagnosis target 10 over the entire width of the oblique part 120 to examine the overall diagnosis target 10 while moving in the longitudinal direction of the oblique part 120.

When the probe unit 130 includes two ultrasound probes 134, the ultrasound probes 134 of the probe unit 130 may be arranged parallel to each other or so as to cross each other in the width direction of the oblique part 120.

When the ultrasound probes 134 are arranged parallel to each other in the width direction of the oblique part 120, the combination of the ultrasound probes 134 can examine the overall diagnosis target 10 as in the case where the probe unit 130 includes a single ultrasound probe 134.

When the ultrasound probes 134 are arranged so as to cross each other in the width direction of the oblique part 120, each of the ultrasound probes 134 can examine the corresponding area of the diagnosis target while moving at different locations.

The ultrasonic diagnostic apparatus 100 according to this embodiment may further include an oblique movement part 140.

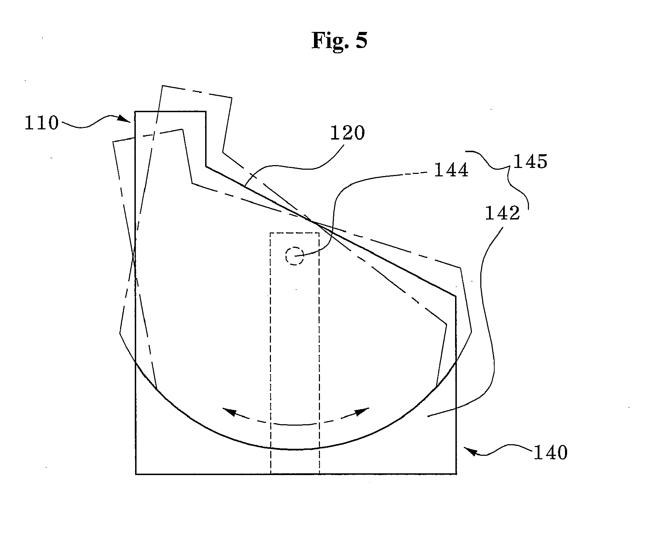

Fig. 5 is a side view of an oblique movement part of the ultrasonic diagnostic apparatus in accordance with the embodiment.

Referring to Fig. 5, the oblique movement part 140 obliquely moves the housing 110 such that the housing 110 approaches or moves away from the diagnosis target 10. The oblique movement part 140 includes a hinge 145 and a slope controller (not shown).

The hinge 145 pivotably couples the housing 110 to the oblique movement part 140, and includes a frame 142 for supporting the housing 110 and a hinge shaft 144 for pivotably coupling the housing 110 to the frame 142.

The slope controller restricts pivoting of the housing 110 around the hinge 145. The slope controller includes a drive motor which generates a drive force for pivoting the housing 110, and a power transmission which transmits the drive force from the drive motor to the housing 110 so that the housing 110 pivots around the hinge 145. The drive motor of the slope controller may be a stepper motor. Such configuration of the slope controller is apparent to a person having ordinary knowledge in the art and a detailed description thereof will be omitted herein.

The housing 110 is obliquely moved by the oblique movement part 140 to approach or move away from the diagnosis target 10 (see Fig. 3), so that the position of the housing 110 can be adjusted depending on the size or shape of the diagnosis target 10.

With the housing 110 obliquely moved away from the diagnosis target 10, the oblique part 120 can stably support the diagnosis target 10 having a large size without shape change of the diagnosis target 10. With the housing obliquely moved to approach the diagnosis target 10, the oblique part 120 can stably support the diagnosis target 10 having a small size without the shape change of the diagnosis target 10.

In this embodiment, the ultrasonic diagnostic apparatus 100 may further include a lift part 150.

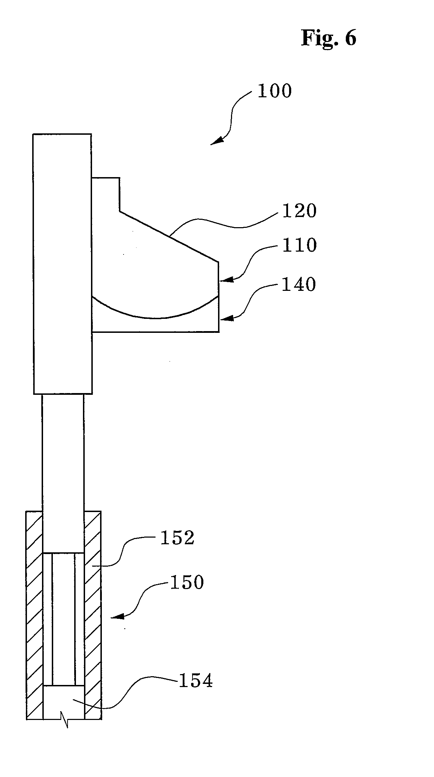

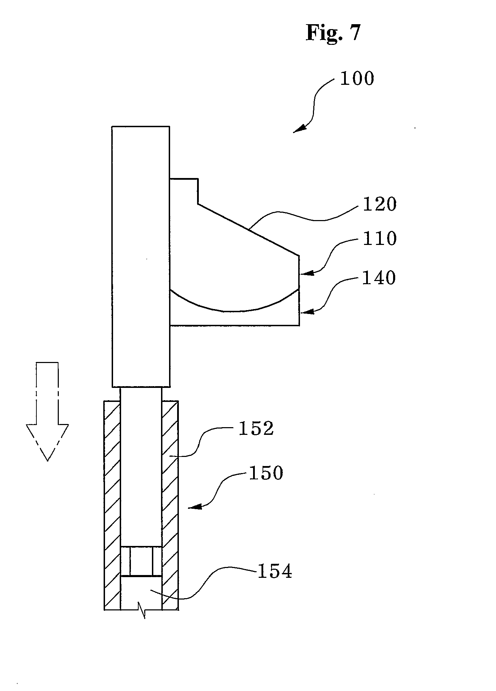

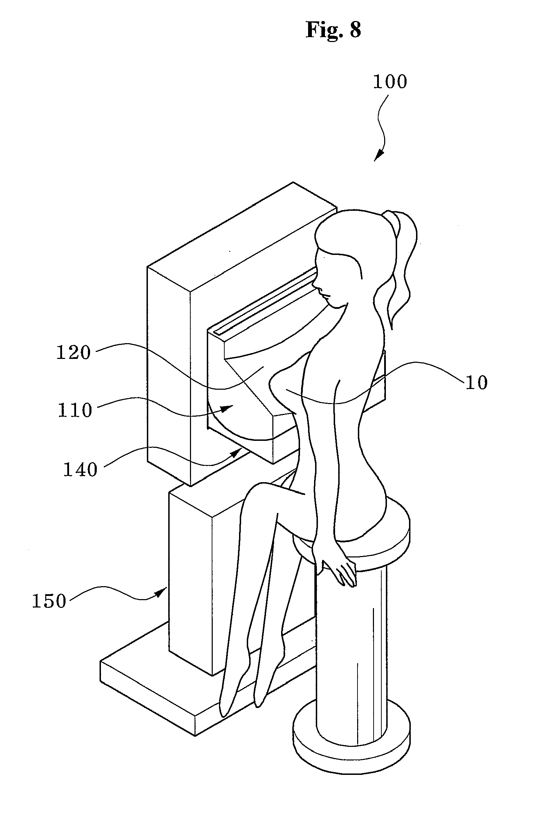

Figs. 6 and 7 are side views of a lift part of the ultrasonic diagnostic apparatus in accordance with the embodiment, and Fig. 8 is a view of another exemplary use of the ultrasonic diagnostic apparatus in accordance with the embodiment.

First, referring to Fig. 6, the lift part 150 raises or lowers the housing 110 and includes a support 152 and a drive unit 154.

The support 152 is disposed below the housing 110 and supports the housing 110 when the housing 110 is raised or lowered by the lift part 150. The support 152 may be coupled to the center or both sides of the housing 110.

The drive unit 154 is disposed in the support 152 and generates a drive force for raising or lowering the housing 110. The drive unit 154 may include an actuator that generates the drive force in the up-down direction to raise or lower the housing 110. Details of the drive unit are apparent to a person having ordinary knowledge in the art and a description thereof will be omitted herein.

As shown in Fig. 7, the lift part 150 raises or lowers the housing 110 to make an upper surface of the oblique part 120 coincident with the height of the diagnosis target 10 so that the diagnosis target 10 can be stably supported by the oblique part 120 not only when an examinee is in a standing posture but also in a sitting posture, as shown in Fig. 8.

Next, operation and effect of the ultrasonic diagnostic apparatus according to this embodiment will be described with reference to Figs. 1 to 8.

When performing ultrasound diagnosis using the ultrasonic diagnostic apparatus 100 according to this embodiment, a diagnosis target 10 of an examinee is supported on the oblique part 120, with the examinee in a standing posture or sitting posture. Here, if the diagnosis target 10 is the breasts of the examinee, it is desirable to manipulate the apparatus such that both breasts of the examinee are supported by the oblique part 120 and substantially most of the breasts come into close contact with the oblique part 120.

For this purpose, as shown in Figs. 6 and 7, the lift part 150 is activated to adjust the upper surface of the oblique part 120 to be positioned at a height coincident with the height of the breasts by raising or lowering the housing 110. At the same time, as shown in Fig. 5, the oblique movement part 140 is activated to adjust the position of the housing 110 to make the shape of the upper surface of the oblique part 120 close to the sizes and shapes of the breasts by obliquely moving the housing 110 to approach or move away from the breasts.

Not only can the housing 110 that is adjustable in height and position as described above stably support both breasts of an examinee, but also allows substantially most of the breasts to come into close contact with the oblique part 120, as shown in Figs. 3 and 4. Thus, the ultrasonic diagnostic apparatus 100 according to this embodiment as shown in Figs. 1 and 2 does not need to excessively compress the breasts in order to allow the breasts to come into close contact with the oblique part 120.

Further, the housing 110 that is adjustable in height and position as above allows the breasts to come into close contact with the oblique part 120 by adjusting the shape of the upper surface of the oblique part 120 to be close to the sizes and shapes of the breasts without compressing the breasts, so that the breasts can come into close contact with the oblique part 120 while maintaining the original shape thereof.

When the diagnosis target 10 is supported on the oblique part 120 as above, ultrasound diagnosis can be performed upon the diagnosis target 10 as shown in Fig. 4.

The ultrasound probe 134 in the housing 110 moves along the guide member 132 that may be formed to include a curved shape, for example, a curved shape corresponding to the longitudinal shape of the oblique part 120. The ultrasound probe 134 examines the diagnosis target 10 while moving along a path including the curved path corresponding to the longitudinal shape of the oblique part 120.

Here, although the diagnosis target 10, that is, both breasts, has a curved shape, the entirety of the diagnosis target 10 is brought into close contact with the oblique part 120 while maintaining the original shape thereof. Thus, the ultrasound probe 134 can obtain continuous images of internal tissue of the breast while reciprocating in the housing 110 and can examine both breasts through a single reciprocation, thereby enabling rapid diagnosis of the diagnosis target 10.

As described above, the ultrasonic diagnostic apparatus 100 according to this embodiment can allow most of the diagnosis target 10 to come into close contact with the oblique part 120 without excessive compression of the diagnosis target 10, thereby enabling efficient diagnosis of the overall diagnosis target 10 without causing discomfort to an examinee due to compression of the diagnosis target 10. Additionally, the ultrasonic diagnostic apparatus 100 allows the diagnosis target 10 to be supported while maintaining the shape thereof, thereby enabling the provision of a constant quality of ultrasound images obtained by repetitious diagnosis while improving reproducibility of diagnosis results.

Furthermore, since the ultrasonic diagnostic apparatus 100 can diagnose the overall diagnosis target 10 through a single diagnosis operation, it is possible to reduce inconvenience of an examinee through rapid diagnosis.

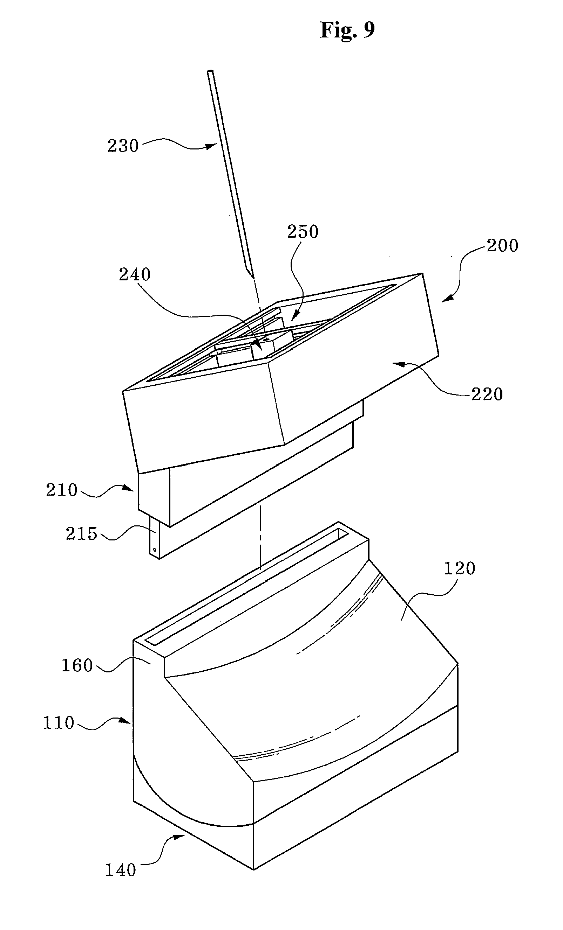

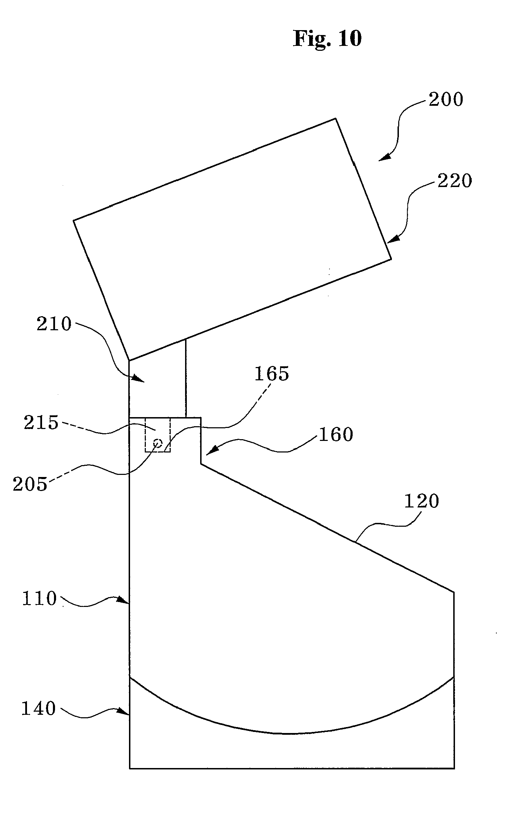

Fig. 9 is an exploded perspective view of the separable biopsy device integrated-type ultrasonic diagnostic apparatus according to the embodiment, and Fig. 10 is a view of a coupling structure between the biopsy device and the ultrasonic diagnostic apparatus shown in Fig. 9.

Referring to Figs. 9 and 10, the biopsy device 200 according to this embodiment is provided to perform a biopsy and is detachably coupled to the ultrasonic diagnostic apparatus 100 at one side of a diagnosis target 10 that is exposed by the ultrasonic diagnostic apparatus 100. The biopsy device 200 includes a second coupling part 210 that is detachably coupled to a first coupling part 160 provided to the ultrasonic diagnostic apparatus 100.

The first coupling part 160 is provided to an upper portion of the ultrasonic diagnostic apparatus 100 and the second coupling part 210 is provide to a lower portion of the biopsy device 200. Further, one of the first and second coupling parts 160 and 210 is formed with a coupling groove 165, and the other is formed with a coupling protrusion 215, which is inserted into the coupling groove 165. In this embodiment, the coupling groove 165 and the coupling protrusion 215 are illustrated as being provided to the first coupling part 160 and the second coupling part 210, respectively, but it should be noted that the invention is not limited thereto.

Furthermore, the separable biopsy device integrated-type ultrasonic diagnostic apparatus 300 may further include a securing part 205. The securing part 205 secures coupling between the first coupling part 160 and the second coupling part 210. For example, the securing part 205 may be composed of, but is not limited to, a combination of a ball spring (reference numeral omitted) provided to the coupling protrusion 215 so as to protrude from the coupling protrusion 215 and a securing groove (reference numeral omitted) into which a portion of the ball spring is inserted.

Generally, biopsy is not performed on all examinees subjected to ultrasound diagnosis using the ultrasonic diagnostic apparatus 100, but only on an examinee suspected of having a tumor according to diagnosis by the ultrasonic diagnostic apparatus 100.

In the separable biopsy device integrated-type ultrasonic diagnostic apparatus 300 according to embodiment that includes the ultrasonic diagnostic apparatus 100 and the biopsy device 200, normally, only ultrasound diagnosis is carried out using only the ultrasonic diagnostic apparatus 100. Then, when there is a need to biopsy an examinee as a follow-up to the ultrasound diagnosis, the biopsy device 200 is coupled to the ultrasonic diagnostic apparatus 100 to perform the biopsy.

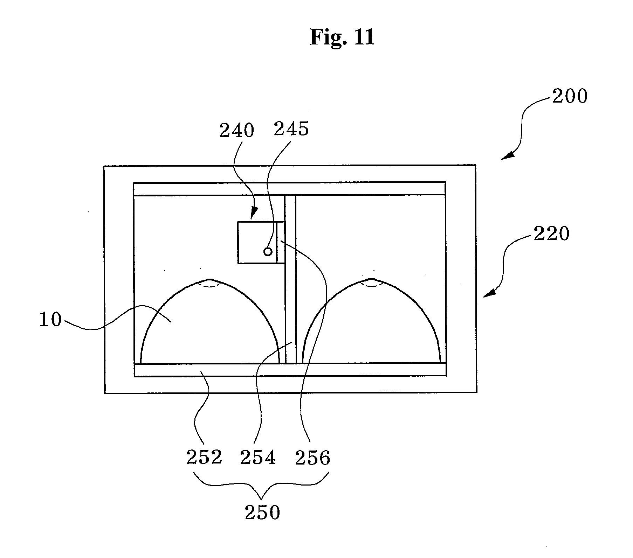

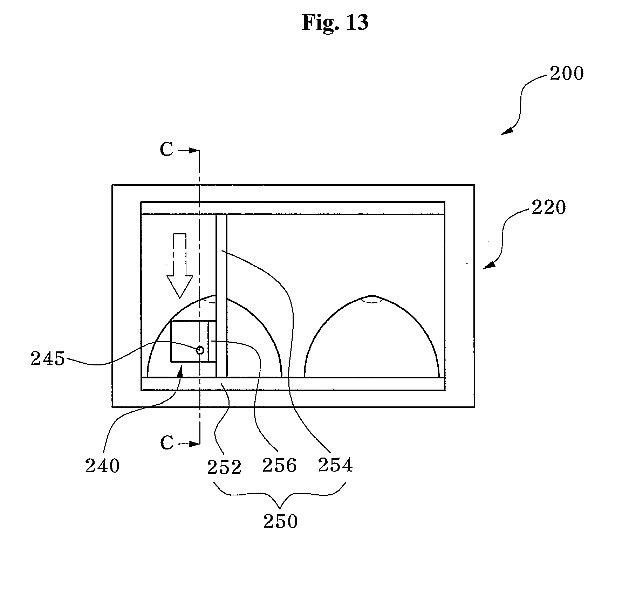

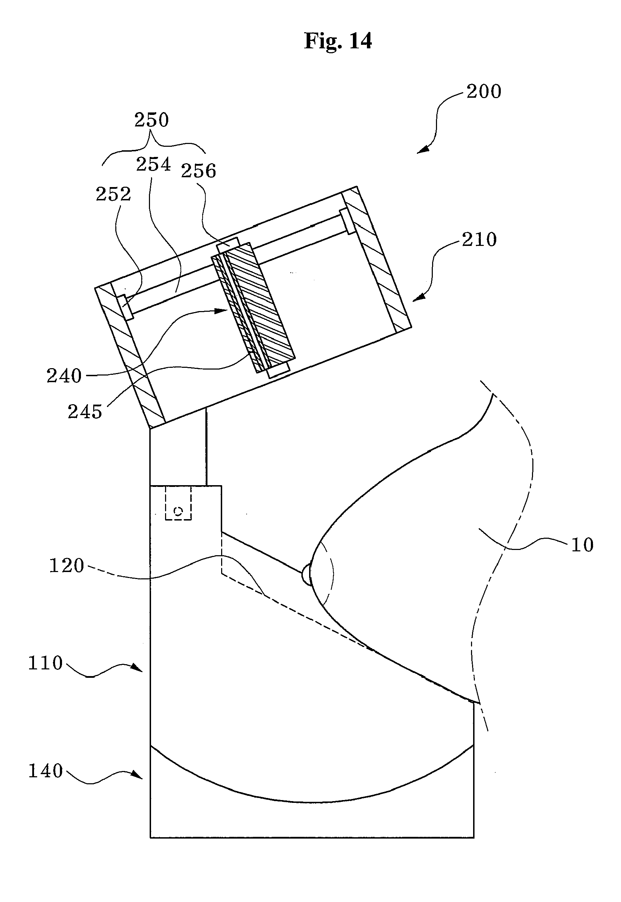

Fig. 11 is a plan view of the biopsy device shown in Fig. 9, Figs. 12 and 13 show an operation of the biopsy device of Fig. 11, Fig. 14 is a cross-sectional view taken along line C-C of Fig. 13, and Figs. 15 and 16 show an operation of a biopsy kit shown in Fig. 14.

First, referring to Figs. 9 to 12, the biopsy device 200 according to this embodiment includes a case 220, a needle 230, a biopsy kit 240, and a biopsy kit-guide 250.

The case 220 forms an outer appearance and preferably has a "□"-shaped cross-section open at upper and lower sides thereof. The case 220 is provided to an upper portion of the second coupling part 210. Preferably, the case 220 may be obliquely provided to the second coupling part 210 to defme an oblique path through which the needle 230 moves.

The needle 230 serves to collect a tissue sample from the diagnosis target 10. The needle 230 has a thin elongated shape and a sharp tip so as to be inserted into the diagnosis target 10. The needle 230 is inserted into the diagnosis target 10 and extracts the tissue sample therefrom.

The biopsy kit 240 is disposed inside the case 220 and is movable leftward, rightward, forward, rearward, upward, and downward. The biopsy kit 240 has a needle guide 245 therein.

The needle guide 245 is formed inside the biopsy kit 240. The needle guide 245 is provided to guide a movement path of the needle 230 and has a guide hole (reference numeral omitted) formed therein to define the movement path of the needle 230. The guide hole formed in the needle guide 245 may have the same or a slightly larger diameter than the needle 230 such that the needle 230 is movably inserted into the guide hole.

The biopsy kit 240 is coupled to the biopsy kit-guide 250. The biopsy kit-guide 250 guides a movement path of the biopsy kit 240 and includes first guide members 252 and a second guide member 254.

The first guide members 252 are disposed in a first direction to guide the biopsy kit 240 in the first direction, and the second guide member 254 is disposed in a different direction from that of the first guide members 252 to guide the biopsy kit 240 in a second direction.

Herein, the first direction is defined as the longitudinal direction of the oblique part 120 (see Fig. 4), that is, the right and left direction of the diagnosis target 10, and the second direction is defined as the width direction of the oblique part 120 (see Fig. 3), that is, the front and backward direction of the diagnosis target 10. Here, considering that the biopsy device 200 according to this embodiment is obliquely disposed at an angle, the first and second directions may be the width direction and the front and backward direction slanted at the oblique angle of the biopsy device 200.

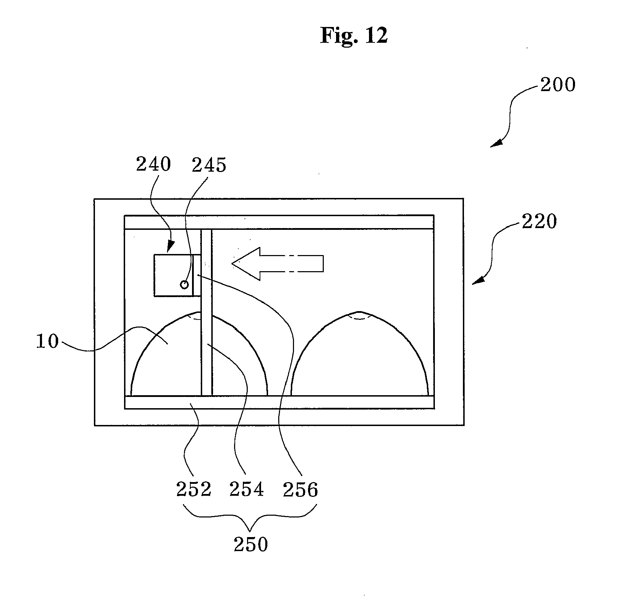

In this embodiment, the first guide members 252 are disposed in the first direction at opposite sides of the case 220. The second guide member 254 is disposed in a different direction than the first guide members 252 and is movably coupled at opposite ends thereof to the first guide members 252 to move in the first direction (see Fig. 12). The second guide member 254 may guide the biopsy kit 240 in the second direction.

Furthermore, as shown in Figs. 11 to 14, the biopsy kit-guide 250 may further include a third guide member 256. The third guide member 256 is disposed in a different direction than the first and second guide members 252 and 254 and guides the biopsy kit 240 in a third direction.

Herein, the third direction is defined as the height direction of the diagnosis target. The third direction may be a height direction slanted at an angle corresponding to the oblique angle of the biopsy device 200 as in the first and second directions.

According to this embodiment, the third guide member 256 is movably coupled to the second guide member 254 to move in the second direction (see Fig. 13). The biopsy kit 240 is movably coupled to the third guide member 256, which can guide the biopsy kit 240 in the third direction.

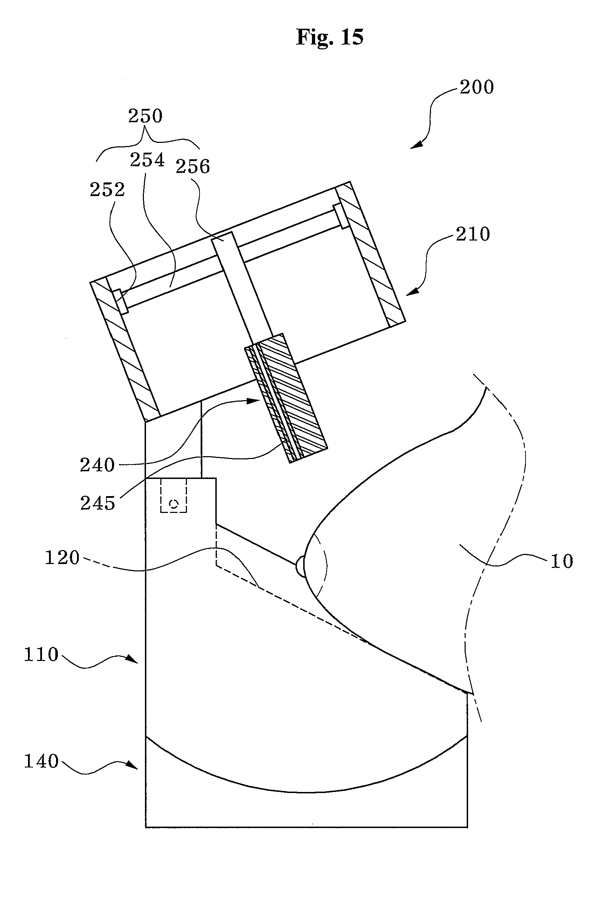

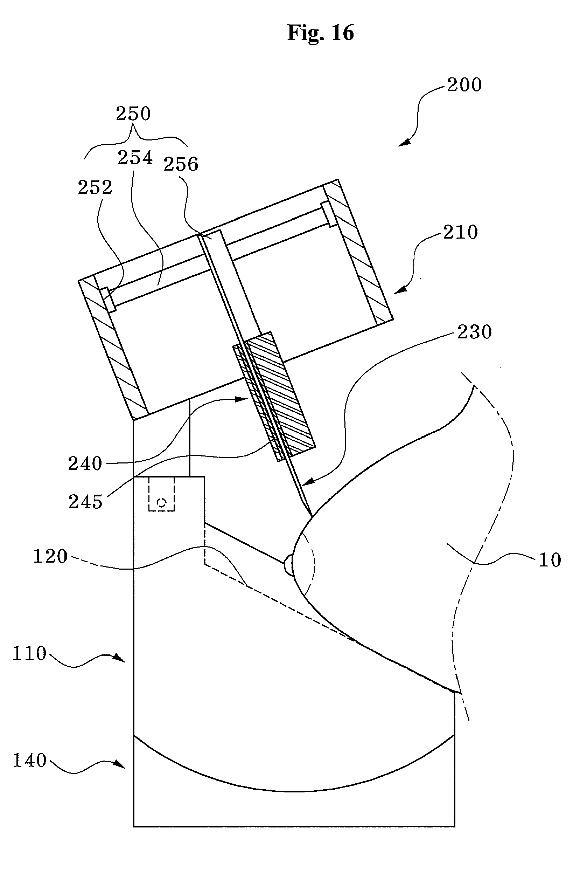

The third guide member 256 guides a movement path of the biopsy kit 240 in the third direction to allow the biopsy kit 240 to approach the diagnosis target 10, so that the height of the biopsy kit 240 may be adjusted (see Fig. 15), and the needle guide 245 of the biopsy kit 240, the height of which is adjusted, guides a movement path of the needle 230, so that the needle 230 may be inserted into the diagnosis target 10 (see Fig. 16).

Next, operation and effect of the biopsy device according to this embodiment will be described.

A biopsy may be performed using the biopsy device 20 after ultrasound diagnosis of the diagnosis target 10 using the ultrasonic diagnostic apparatus 100 or may be performed independent of the ultrasound diagnosis.

When the biopsy is performed after the ultrasound diagnosis, the biopsy device 200 is coupled to the ultrasound diagnostic apparatus 100 with the diagnosis target 10 supported by the oblique part 120, as shown in Fig. 14.

When performed independent of the ultrasound diagnosis, the biopsy may be performed after coupling the biopsy device 200 to the ultrasound diagnosis apparatus 100 with the diagnosis target 10 supported by the oblique part 120, or after supporting the diagnosis target 10 by the oblique part 120 with the biopsy device 200 coupled to the ultrasound diagnosis apparatus 100.

Then, front, back, right and left positions of the biopsy kit 240 are adjusted by moving the biopsy kit 240 in the first and second directions, as shown in Figs. 11 and 13. Additionally, upper and lower positions of the biopsy kit 240 are adjusted by moving the biopsy kit 240 in the third direction as shown in Fig. 15, thereby allowing the needle guide 245 of the biopsy kit 240 to approach the diagnosis target 10.

Such movement of the biopsy kit 240 may be manually obtained by manipulation of a user. Alternatively, the movement of the biopsy kit 240 may be automatically obtained by a controller (not shown) controlling the overall operation of the separable biopsy device integrated-type ultrasonic diagnostic apparatus 300, a drive unit (not shown) controlled by the controller to generate a drive force for moving the second guide member 254, third guide member 256 and biopsy kit 240, and a power transmission (not shown) transmitting the drive force from the drive unit to the second guide member 254, third guide member 256 and biopsy kit 240.

Moreover, when a portion of the diagnosis target 10 suspected of having a tumor based on ultrasound images obtained by ultrasound diagnosis performed prior to the biopsy, that is, a target position to be biopsied, is determined, the controller adjusts the position of the biopsy kit 240 by controlling the drive unit using such information to thereby allow the needle guide 245 to be precisely positioned at the target position.

Next, the needle 230 is moved through the guide hole in the needle guide 245 and inserted into the diagnosis target 10, as shown in Fig. 16.

As apparent from the above description, the separable biopsy device integrated-type ultrasonic diagnostic apparatus 300 according to this embodiment is configured to perform ultrasound diagnosis and biopsy together, thereby providing the convenience of allowing the ultrasound diagnosis and the biopsy to be performed with a single apparatus. Further, the apparatus 300 can perform a biopsy based on ultrasound images obtained using the ultrasonic diagnostic apparatus 100 without changing a position of the diagnosis target 10, thereby enabling precise determination as to a position to be biopsied, that is, a target position into which the needle 200 of the biopsy device will be inserted.

Further, according to the embodiment, the separable biopsy device integrated-type ultrasonic diagnostic apparatus 300 allows the biopsy device 200 to be separated and stored separately from the ultrasonic diagnostic apparatus 100 when not in use, thereby reducing a space occupied by the entire apparatus and preventing damage of the biopsy device 200 due to disuse of the biopsy device 200 for a long time in a state of being coupled to the ultrasonic diagnostic apparatus 100.

Although some embodiments have been provided to illustrate the invention in conjunction with the drawings, it will be apparent to those skilled in the art that the embodiments are given by way of illustration only, and that various modifications and equivalent embodiments can be made without departing from the spirit and scope of the invention. The scope of the invention should be limited only by the accompanying claims.

## Claims

1. A separable biopsy device integrated-type ultrasonic diagnostic apparatus (300), **characterized by** comprising:
an ultrasonic diagnostic apparatus (100) exposing one side of a diagnosis target (10) and examining the diagnosis target (10) at the other side of the diagnosis target (10); and
a biopsy device (200) located at the exposed side of the diagnosis target (10) and detachably coupled to the ultrasonic diagnostic apparatus (100).

2. The apparatus (300) according to claim 1, **characterized by** further comprising:
a first coupling part (160) provided to the ultrasonic diagnostic apparatus (100); and
a second coupling part (210) provided to the biopsy device (200) to be detachably coupled to the first coupling part (160).

3. The apparatus (300) according to claim 2, **characterized in that** one of the first and second coupling parts (160, 210) is formed with a coupling groove (165), and the other is formed with a coupling protrusion (215) inserted into the coupling groove (165).

4. The apparatus (300) according to claim 3, **characterized by** further comprising:
a securing part (205) that secures coupling between the first coupling part (160) and the second coupling part (210).

5. The apparatus (300) according to claim 1, **characterized in that** the biopsy device (200) comprises a needle (230) collecting a tissue sample from the diagnosis target (10), a biopsy kit (240) having a needle guide (245) guiding a movement path of the needle (230), and a biopsy kit-guide (250) coupled with the biopsy kit (240) and guiding a movement path of the biopsy kit (240).

6. The apparatus (300) according to claim 5, **characterized in that** the biopsy kit-guide (250) comprises a first guide member (252) disposed in a first direction to guide the biopsy kit (240) in the first direction, and a second guide member (254) disposed in a different direction from that of the first guide member (252) to guide the biopsy (240) kit in a second direction.

7. The apparatus (300) according to any one of claims 1 to 6, **characterized in that** the ultrasonic diagnostic apparatus (100) comprises a housing (110) exposing one side of the diagnosis target (10) while supporting the diagnosis target (10) so as to correct a shape of the diagnosis target (10), an oblique part (120) obliquely formed on the housing (110) to support the diagnosis target (10), and a probe unit (130) disposed inside the housing (110) to examine the diagnosis target (10).

8. The apparatus (300) according to claim 7, **characterized in that** the oblique part (120) has an upward slope corresponding to the shape of the diagnosis target (10) from a lower side of the oblique part (120) to an upper side thereof.

9. The apparatus (300) according to claim 7, **characterized in that** the diagnosis target (10) is a breast of an examinee, the oblique part (120) has a length supporting both breasts of the examinee, and the probe unit (130) moves along a path including a curved path to examine both breasts of the examinee through a single examination operation.

10. The apparatus (300) according to claim 7, **characterized by** further comprising: a lift part (150) raising or lowering the housing (110).

11. The apparatus (300) according to claim 7, **characterized by** further comprising:
an oblique movement part (140) obliquely moving the housing (110) to approach or move away from the diagnosis target (10).

12. The apparatus (300) according to claim 1, **characterized in that** the ultrasonic diagnostic apparatus (100) is an upright type ultrasonic diagnostic apparatus.
